# EUROPÄISCHE PATENTSCHRIFT

(11) **EP 0 672 731 B1**
(45) Veröffentlichungstag und Bekanntmachung des Hinweises auf die Patenterteilung: **05.06.1996**
(21) Anmeldenummer: 94118099.4
(22) Anmeldetag: 17.11.1994
(51) Int. Cl.: C09C 1/30

(54) **Silanisierte Kieselsäuren**
Silanised silicas
Silices silanisées

(30) Priorität: 27.01.1994 DE 4402370
(43) Veröffentlichungstag der Anmeldung: 20.09.1995
(73) Patentinhaber: Degussa Aktiengesellschaft, D-60311 Frankfurt (DE)
(72) Erfinder: Ettlinger, Manfred, Dr., D-63791 Karlstein (DE); Kerner, Dieter, Dr., D-63450 Hanau (DE); Meyer, Jürgen, Dr., D-79618 Rheinfelden (DE)

(56) Entgegenhaltungen:
- EP-A- 0 216 047
- EP-A- 0 475 132
- PATENT ABSTRACTS OF JAPAN vol. 12, no. 258 (C-513) 20. Juli 1988 & JP-A-63 043 976 (ASAHI CHEM. IND. CO.) 25. Februar 1988
- WORLD SURFACE COATINGS ABSTRACTS, Bd.64, Nr.583, 1991, OXFORD GB

## Beschreibung

Die Erfindung betrifft silanisierte Kieselsäuren, das Verfahren zu ihrer Herstellung sowie ihre Verwendung als Verdickungsmittel.

Es ist bekannt, eine silanisierte, pyrogen hergestellte Kieselsäure herzustellen, indem man die pyrogen hergestellte Kieselsäure mit Dimethyldichlorsilan behandelt (DE-AS 11 63 784).

Weiterhin sind pyrogen hergestellte Kieselsäuren bekannt, die an der Oberfläche chemisch gebundene -SiC₈H₁₇-Gruppen, Trimethylsilylgruppen oder Polydimethylsiloxangruppen tragen (Schriftenreihe Pigmente Nr. 11, Seite 15, Ausgabe August 1991).

Ferner ist aüs _{"}World Surface Coatings Abstracts", Bd. 64, 583, 1991, Nr. 91/00002 eine Fällüngs-Kieselsäure bekannt, die mit Octadecyl-Trimethoxy-Silan modifiziert ist.

Gegenstand der Erfindung sind silanisierte, pyrogen hergestellte Kieselsäuren, welche dadurch gekennzeichnet sind, daß die pyrogen hergestellten Kieselsäuren mit einer Verbindung aus der Gruppe (RO)₃SiCₙH₂ₙ₊₁, wobei n = 10 bis 18 und R = kürzkettige Alkyl-, wie zum Beispiel Methyl-, oder Ethyl- Reste bedeuten, behandelt sind.

Als pyrogen hergestellte Kieselsäure kann eine auf hochtemperaturhydrolytischem Wege aus SiCl₄ + H₂ und O₂ hergestellte Kieselsäure verwendet werden.

Insbesondere kann eine temperaturhydrolytisch hergestellte Kieselsäure eingesetzt werden, die die folgenden physikalisch-chemischen Kenndaten aufweist:

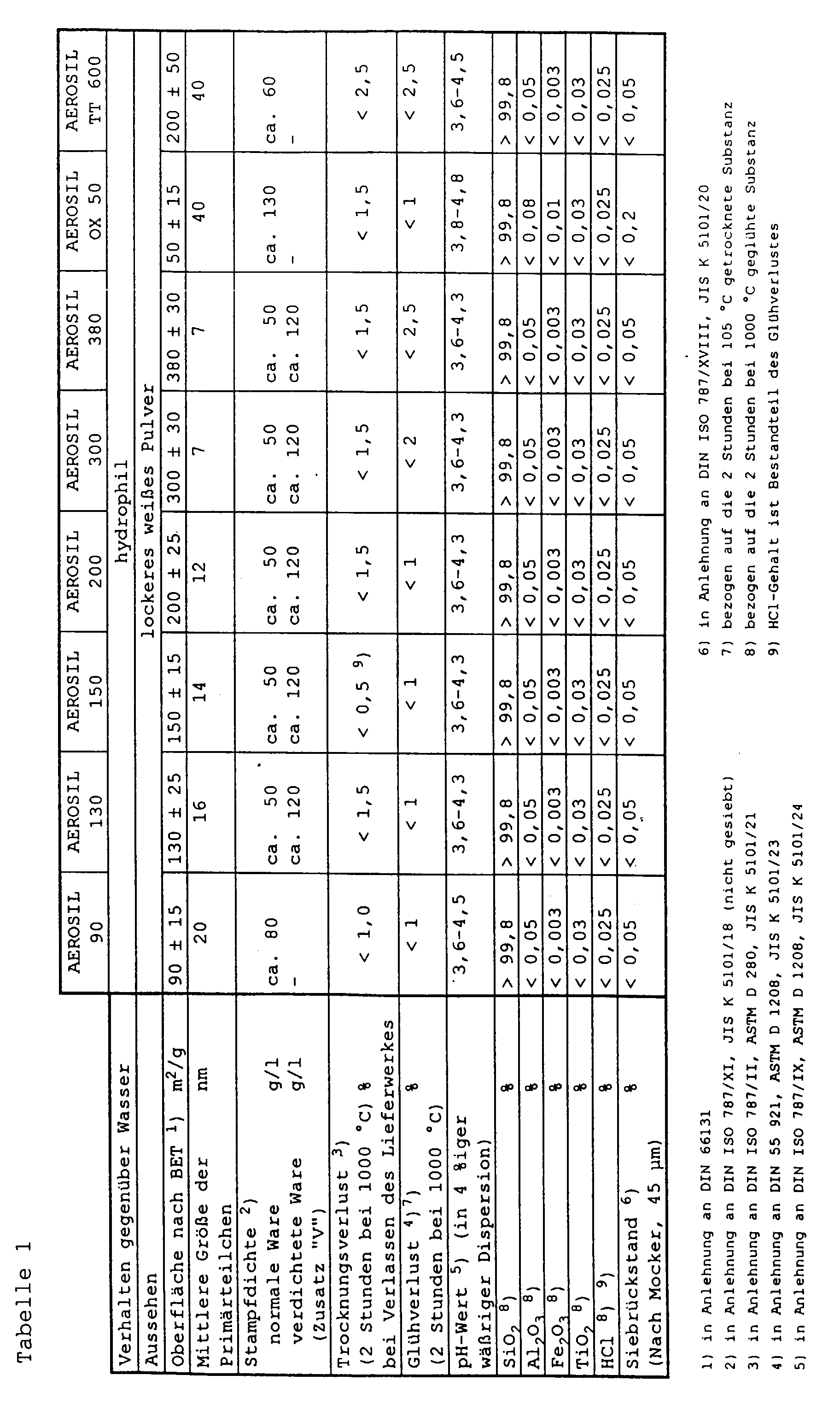

Derartige pyrogene Kieselsäuren sind bekannt. Sie werden unter anderem beschrieben in:
Winnacker-Küchler, Chemische Technologie, Band 3 (1983), 4. Auflage, Seite 77 und
Ullmanns Encyklopädie der technischen Chemie, 4. Auflage (1982), Band 21, Seite 462.

Die pyrogen hergestellten Kieselsäuren werden mit einer Verbindung aus der Gruppe (RO)₃SiCₙH₂ₙ₊₁, wobei n = 10 bis 18 und R = kurzkettige Alkyl-, wie zum Beispiel Methyl-, oder Ethyl- Reste bedeuten, behandelt.

Insbesondere können die folgenden Verbindungen eingesetzt werden:
Silan I (CH₃O)₃SiC₁₆H₃₃ (Hexadecyltrimethoxysilan)
Silan II (CH₃O)₃SiC₁₈H₃₇ (Octadecyltrimethoxysilan)

Die erfindungsgemäßen Kieselsäuren können hergestellt werden, indem man die pyrogen hergestellten Kieselsäuren in einen Mischer vorlegt, unter intensivem Mischen die Kieselsäuren gegebenenfalls zunächst mit Wasser und anschließend mit der Verbindung (Organosilan) aus der Gruppe (RO)₃SiCₙH₂ₙ₊₁ besprüht, 15 bis 30 Minuten nachmischt und anschließend bei einer Temperatur von 100 bis 160 °C über einen Zeitraum von 1 bis 3 Stunden tempert.

Das eingesetzte Wasser kann mit einer Säure, zum Beispiel Salzsäure, bis zu einem pH-Wert von 7 bis 1 angesäuert sein.

Das eingesetzte Organosilan kann in einem Lösungsmittel, wie zum Beispiel Ethanol, gelöst sein.

Die Temperung kann in einer Schutzgasatmosphäre, wie zum Beispiel unter Stickstoff, durchgeführt werden.

Die erfindungsgemäßen, mit Silan I silanisierten, pyrogen hergestellten Kieselsäuren weisen die in Tabelle 2 aufgeführten physikalisch-chemischen Kenndaten auf:

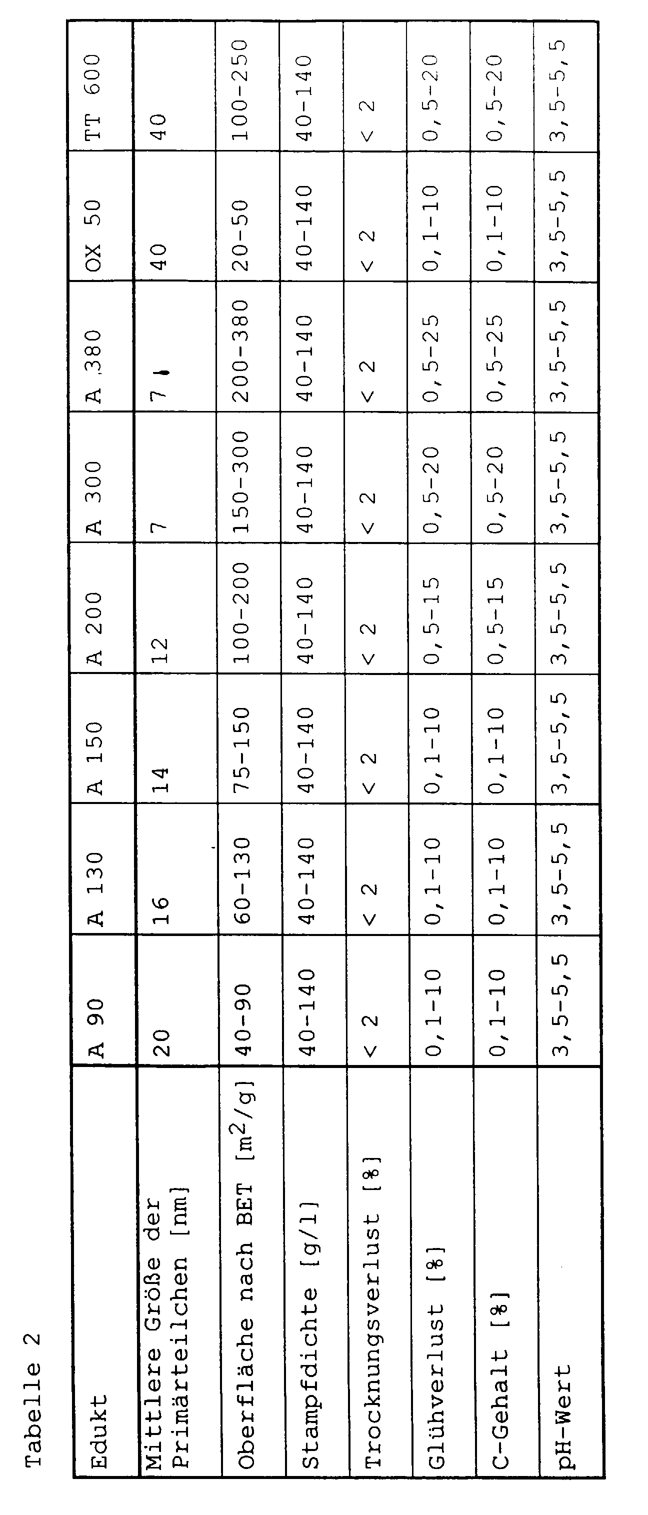

Die erfindungsgemäßen Kieselsäuren können als Verdickungsmittel in Flüssigkeiten, wie wasserverdünnbare Lacke, und Harze, wie zum Beispiel Epoxyharze, eingesetzt werden. Weiterhin können die erfindungsgemäßen Kieselsäuren in Silikonkautschuk, Gummi, Kosmetikartikel, Tonerpulvern sowohl als Mittel zur Verbesserung der Rieselfähigkeit als auch als Verstärkerfullstoff eingesetzt werden.

### Beispiele

Die eingesetzten, pyrogen hergestellten Kieselsäuren weisen die physikalisch-chemischen Kenndaten, die in der Tabelle 1 aufgeführt sind, auf.

Als Organosilane werden die folgenden Verbindungen der allgemeinen Formel (RO)₃SiCₙH₂ₙ₊₁ eingesetzt:
(Silan I) (CH₃O)₃SiC₁₆H₃₃
(Silan II) (CH₃O)₃SiC₁₈H₃₇

Die Kieselsäure wird in einem Mischer vorgelegt und unter intensivem Mischen zunächst mit Wasser und anschließend mit Organosilan besprüht.

Nachdem das Besprühen beendet ist, wird noch 15 bis 30 Minuten nachgemischt und anschließend 1 bis 3 Stunden bei 100 bis 160 °C getempert. Die Temperung kann auch unter Schutzgas, zum Beispiel Stickstoff, erfolgen.

Die einzelnen Reaktionsbedingungen können der Tabelle 3 entnommen werden.

Die physikalisch-chemischen Kenndaten der erhaltenen silanisierten Kieselsäuren sind in der Tabelle 3 bis 4 aufgeführt.

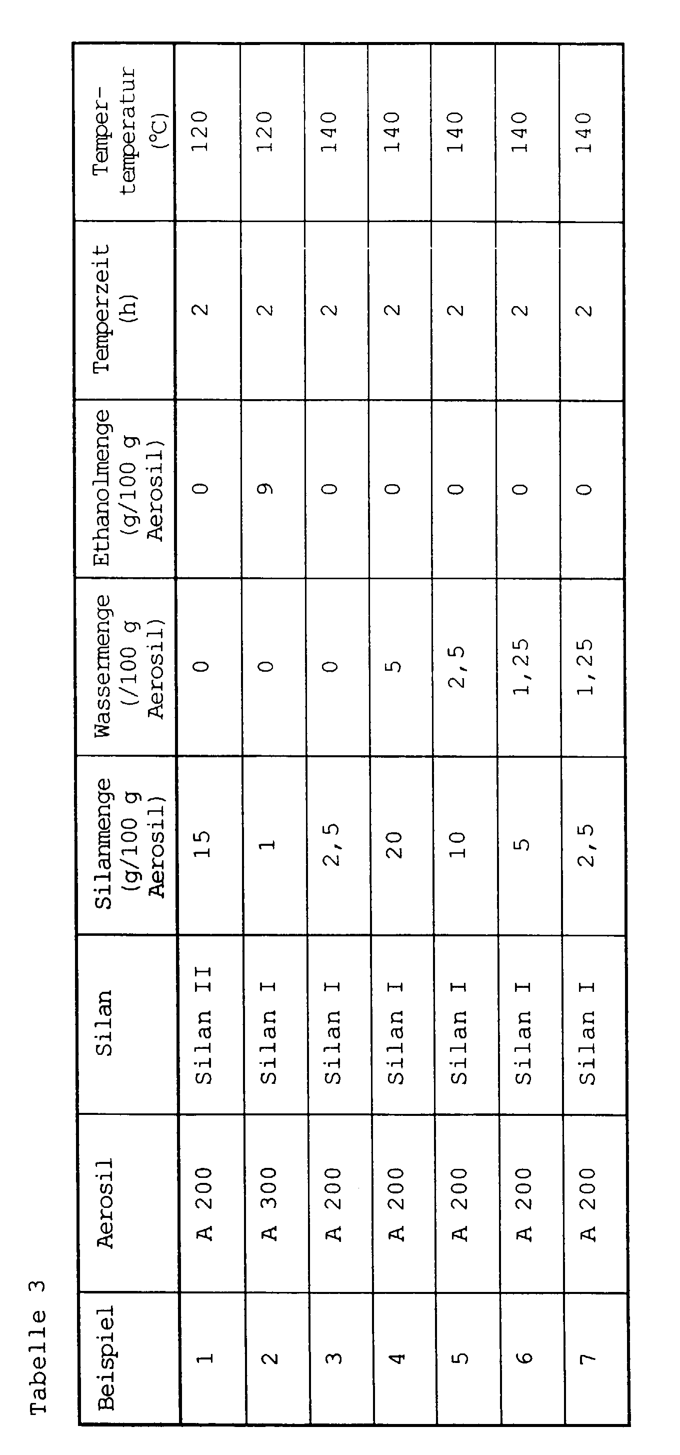

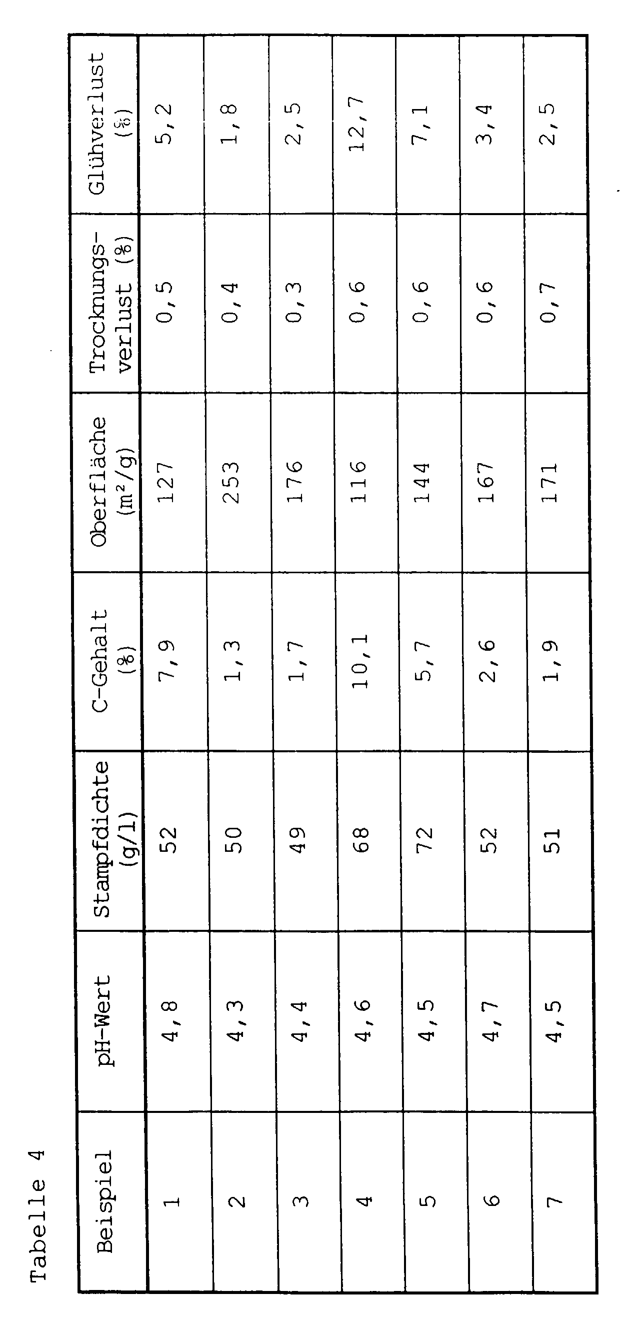

An den erfindungsgemäß hergestellten Kieselsäuren wird die Verdickungswirkung untersucht. Als Modellsystem wird ein Propanol/Wasser-Gemisch 1 : 1 gewählt, 150 g Ansätze, Einwaage 7,5 g.Kieselsäure (5 Gew.-%). 5 Minuten bei 2500 U/min mit Disolver dispergiert und mit Brookfield-Viskosimeter RVT (Spindel 4) gemessen:

| Beispiel | System bzw. Kieselsäure | Viskosität |
|---|---|---|
| 8 | Propanol/Wasser 1:1 | 80 |
| 9 | Aerosil 200 | 200 |
| 10 | gemäß Beispiel 3 | 400 |
| 11 | gemäß Beispiel 4 | 14000 |
| 12 | gemäß Beispiel 5 | 9800 |
| 13 | gemäß Beispiel 6 | 800 |
| 14 | gemäß Beispiel 7 | 400 |

Es ist ersichtlich, daß die erfindungsgemäßen silanisierten Kieselsäuren bezüglich Verdickung der unbehandelten Ausgangskieselsäure A 200 überlegen sind.

## Patentansprüche

1. Silanisierte, pyrogen hergestellte Kieselsäuren, dadurch gekennzeichnet, daß die pyrogen hergestellten Kieselsäuren mit einer Verbindung aus der Gruppe (RO)₃SiCₙH₂ₙ₊₁, wobei n = 10 bis 18 und R = kurzkettige Alkyl-Reste bedeuten, behandelt sind.

2. Silanisierte, pyrogen hergestellte Kieselsäuren gemäß Anspruch 1, dadurch gekennzeichnet, daß die pyrogen hergstellten Kieselsäuren mit der Verbindung (CH₃O)₃SiC₁₆H₃₃ (Hexadecyltrimethoxysilan) behandelt wurden.

3. Silanisierte, pyrogen hergestellte Kieselsäuren gemäß Anspruch 1, dadurch gekennzeichnet, daß die pyrogen hergestellten Kieselsäuren mit der Verbindung (CH₃O)₃SiC₁₈H₃₇ (Octadecyltrimethoxysilan) behandelt wurden.

4. Verfahren zur Herstellung der silanisierten, pyrogen hergestellten Kieselsäuren gemäß den Ansprüchen 1 bis 3, dadurch gekennzeichnet, daß man die pyrogen hergestellten Kieselsäuren in einem Mischer vorlegt, unter intensivem Mischen die Kieselsäuren, gegebenenfalls zunächst mit Wasser und anschließend mit der Verbindung aus der Gruppe (RO)₃SiCₙH₂ₙ₊₁ besprüht, 15 bis 30 Minuten nachmischt und anschließend bei einer Temperatur von 100 bis 160 °C über einen Zeitraum von 1 bis 3 Stunden tempert.

5. Verwendung der silanisierten, pyrogen hergestellten Kieselsäuren zum Verdicken von Flüssigkeiten.

## Claims

1. Silanised, pyrogenically prepared silicas, characterised in that the pyrogenically prepared silicas are treated with a compound from the group (RO)₃SiCₙH₂ₙ₊₁, wherein n equals 10 to 18 and R signifies short-chain alkyl radicals.

2. Silanised, pyrogenically prepared silicas according to claim 1, characterised in that the pyrogenically prepared silicas were treated with the compound (CH₃O)₃Si₁₆H₃₃ (hexadecyltrimethoxysilane).

3. Silanised, pyrogenically prepared silicas according to claim 1, characterised in that the pyrogenically prepared silicas were treated with the compound (CH₃O)₃Si₁₈H₃₇ (octadecyltrimethoxysilane).

4. Method for the preparation of the silanised, pyrogenically prepared silicas according to claims 1 to 3, characterised in that the pyrogenically prepared silicas are placed in a mixer, with intimate mixing the silicas are sprayed, optionally first with water and subsequently with the compound from the group (RO)₃SiCₙH₂ₙ₊₁, are remixed for 15 to 30 minutes and subsequently tempered for a period of from 1 to 3 hours at a temperature of from 100 to 160°C.

5. Use of the silanised, pyrogenically prepared silicas for thickening liquids.

## Revendications

1. Acides siliciques silanisés, préparés par pyrogénation, caractérisés en ce que les acides siliciques préparés par pyrogénation ont été traités avec un composé du groupe (RO)₃SiCₙH₂ₙ₊₁, dans lequel n = 10 à 18 et R représente des radicaux alkyle à chaîne courte.

2. Acides siliciques silanisés, produits par pyrogénation selon la revendication 1, caractérisés en ce que les acides siliciques préparés par pyrogénation ont été traités avec le composé (CH₃O)₃SiC₁₆H₃₃ (hexadécyltriméthoxysilane).

3. Acides siliciques silanisés, produits par pyrogénation selon la revendication 1, caractérisés en ce que les acides siliciques préparés par pyrogénation ont été traités avec le composé (CH₃O)₃SiC₁₈H₃₇ (octadécyltriméthoxysilane).

4. Procédé de préparation des acides siliciques silanisés, produits par pyrogénation selon les revendications 1 à 3, caractérisé en ce qu'on dépose les acides siliciques produits par pyrogénation dans un mélangeur, qu'on asperge en mélangeant intensément les acides siliciques, éventuellement d'abord avec de l'eau et ensuite avec le composé du groupe (RO)₃SiCₙH₂ₙ₊₁, qu'on mélange encore pendant 15 à 30 minutes et ensuite qu'on équilibre la température de 100 à 160°C pendant une durée de 1 à 3 heures.

5. Utilisation des acides siliciques silanisés, produits par pyrogénation pour l'épaississement de liquides.
